# EUROPEAN PATENT APPLICATION

(11) **EP 4 462 442 A1**
(43) Date of publication of application: **13.11.2024**
(21) Application number: 23172792.6
(22) Date of filing: 11.05.2023
(51) Int. Cl.: G16H 40/40, G16H 40/60, A61B 6/00

(54) **EVALUATING A PERFORMANCE METRIC FOR A COMPONENT OF A MEDICAL IMAGING SYSTEM**

(71) Applicant: Koninklijke Philips N.V., 5656 AG Eindhoven (NL)
(72) Inventor: VOGTMEIER, Gereon, Eindhoven (NL); FORTHMANN, Peter, Eindhoven (NL); RIBBING, Carolina Maria, 5656AG Eindhoven (NL); DOUGLAS, Alexander Ulrich, Eindhoven (NL)
(74) Representative: Philips Intellectual Property & Standards

(57) **Abstract**

A computer-implemented method of evaluating a performance metric for a component of a medical imaging system, is provided. The method includes acquiring monitoring data for the component during a plurality of separate time intervals (180_{1..i}) within a monitoring period (140₁). Each time interval is prior to a performance of a diagnostic imaging scan (190) by the medical imaging system, or subsequent to a performance of a diagnostic imaging scan by the medical imaging system. The value of the performance metric is calculated based on the values of the set of monitored parameters acquired during the monitoring period. A revised set of monitored parameters, or a revised set of measurement conditions, is used to acquire monitoring data during one or more subsequent monitoring periods (140₂, 140₃), and the performance metric is re-calculated. The revised set of monitored parameters, or the revised set of measurement conditions, is determined based on a rate of change of a value of one or more of the monitored parameters during a preceding monitoring period.

## Description

### FIELD OF THE INVENTION

The present disclosure relates to evaluating a performance metric for a component of a medical imaging system. A computer-implemented method, a computer program product, and a system, are disclosed.

### BACKGROUND OF THE INVENTION

Medical imaging systems such as X-ray imaging systems, computed tomography, "CT", imaging systems, magnetic resonance imaging, "MRI", systems, and so forth, may occasionally malfunction. A minor malfunction may permit a continuation of imaging procedures using the medical imaging system. A more severe malfunction may require a suspension of imaging procedures. In both cases, a malfunction can interrupt workflow whilst the cause of the malfunction is investigated.

In order to reduce the likelihood of such malfunctions, and also to assist their investigation, medical imaging systems typically acquire monitoring data for their components. The monitoring data is also known as log data, and may be recorded in a so-called "log file". The log files of medical imaging systems are sometimes analysed in a proactive manner in order to try to predict potential problems with their components. This involves intermittently evaluating various performance metrics for the components of the medical imaging system in advance of their anticipated failure. The performance metrics that are evaluated may represent factors such as an expected remaining lifetime of the component, a potential fault with the component, an amount of degradation of the component, and so forth. Such performance metrics may be used to schedule maintenance operations on a component in a timely manner by, for instance, replacing, or repairing, the relevant component. The log files of medical imaging systems are also sometimes analysed in a reactive manner. This involves analysing the log files after a malfunction has occurred in order to try to identify its cause.

Initially, the log files that are generated by medical imaging systems may be stored locally with respect to the imaging system. For example, they may be stored on a computer, or on a server, that is connected to the imaging system and located at the same medical facility. Subsequently, the log files may be transmitted over a communication network to a remote location. The transmission of the log data to the remote location may be performed periodically (e.g. on a daily, or weekly basis), or on an ad-hoc basis (e.g. depending on factors such as connectivity to the communication network, and usage of local data storage capacity). The analysis of the log data, in both a proactive manner, and a reactive manner, may be performed locally with respect to the imaging system, or it may be performed at the remote location. However, medical imaging systems can generate a significant amount of log data, and their size presents challenges in terms of data storage requirements, and also in terms of communication bandwidth requirements in situations in which the data is transmitted to a remote location.

A further challenge for the proactive manner of operation described above is the need to acquire data that is relevant to the performance metrics that are evaluated. In principle, numerous parameters might provide useful information for evaluating each performance metric. However, the acquisition of data for all such potentially relevant parameters is burdensome in terms of its data storage requirements and its communication bandwidth requirements. It may therefore only be practical to acquire a limited amount of monitoring data. This limits the accuracy of the performance metrics that are evaluated.

Thus, there remains a need to improve the acquisition of monitoring data for evaluating performance metrics of medical imaging systems.

### SUMMARY OF THE INVENTION

According to one aspect of the present disclosure, a computer-implemented method of evaluating a performance metric for a component of a medical imaging system, is provided. The method includes:
- acquiring monitoring data for the component during a monitoring period;
- wherein the monitoring data comprises values of a set of monitored parameters, and wherein the values of the set of monitored parameters are acquired under a corresponding set of measurement conditions; and
- wherein the monitoring data is acquired during a plurality of separate time intervals within the monitoring period, each time interval being prior to a performance of a diagnostic imaging scan by the medical imaging system, or subsequent to a performance of a diagnostic imaging scan by the medical imaging system.

The method also includes:
- calculating the value of the performance metric for the component based on the values of the set of monitored parameters acquired during the monitoring period; and
- repeating the acquiring, and the calculating, for monitoring data acquired during one or more subsequent monitoring periods, and wherein the monitoring data acquired during the one or more subsequent monitoring periods is acquired using a revised set of monitored parameters, or a revised set of measurement conditions; and
- wherein the revised set of monitored parameters, or the revised set of measurement conditions, used to acquire the monitoring data during the one or more subsequent monitoring periods is determined based on a rate of change of a value of one or more of the monitored parameters during a preceding monitoring period.

In the above method, monitoring data is acquired during time intervals that are prior to a performance of a diagnostic imaging scan by the medical imaging system, or subsequent to a performance of a diagnostic imaging scan by the medical imaging system. The use of such time periods avoids interruptions to a diagnostic imaging scan. Moreover, the use of such time periods facilitates the acquisition of monitoring data under measurement conditions that are difficult, or even impossible, to provide during a diagnostic imaging scan due to their potential impact on the scan. Thus, it facilitates access to a wider range of measurement conditions. This wider range of measurement conditions can result in observations that are either not apparent, or entirely absent, from measurement data that is acquired data during a scan. Thus, it helps to elucidate subtle changes in the performance of a component.

In the above method, the revised set of monitored parameters, or the revised set of measurement conditions, that is used to acquire the monitoring data during the one or more subsequent monitoring periods, is determined based on a rate of change of a value of one or more of the monitored parameters during a preceding monitoring period. An insight of the inventors that is exploited in this operation, is that monitored parameters, or measurement conditions, that give rise to monitored parameter values that undergo relatively smaller changes over time have relatively less impact on the calculated value of the performance metric than monitored parameter values that undergo relatively larger changes over time. Revising the set of monitored parameters, or the revised set of measurement conditions, based on a rate of change of a value of one or more of the monitored parameters, facilitates the acquisition of data from monitored parameters, or under measurement conditions, that has a greater impact on the performance metric. It also, helps to limit the data storage and transmission requirements of the monitoring data. For instance, monitored parameters that give rise to monitored parameter values that undergo relatively smaller changes over time, can be removed from the set, or measured at relatively larger time intervals. Similarly, measurement conditions that give rise to monitored parameter values that undergo relatively smaller changes over time, can be adjusted in order to provide monitored parameter values that undergo relatively larger changes over time. In other words, the method provides for the acquisition of monitoring data that has greater relevance to the performance metric. Thus, it improves the accuracy of the performance metric, and also alleviates the data storage requirements, and the communication bandwidth requirements, of storing, and transmitting, the monitoring data.
Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a computer-implemented method of evaluating a performance metric for a component of a medical imaging system, in accordance with some aspects of the present disclosure.
Fig. 2 is a schematic diagram illustrating an example of a system 200 for evaluating a performance metric for a component 110 of a medical imaging system 120, in accordance with some aspects of the present disclosure.
Fig. 3 illustrates an example of monitoring data 130 that is acquired for an X-ray tube of a CT imaging system, in accordance with some aspects of the present disclosure.
Fig. 4 is an example illustrating the acquisition of monitoring data during a plurality of separate time intervals 180_{1..i} within a monitoring period 140₁, and wherein each time interval is prior to a performance of a diagnostic imaging scan 190 by a medical imaging system, or subsequent to a performance of a diagnostic imaging scan by the medical imaging system, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a computer-implemented method that involves evaluating a performance metric for a component of a medical imaging system. Reference is made to examples in which the component is an X-ray tube of a CT imaging system. However, it is to be appreciated that the method may be used to evaluate performance metrics for other types of components in a CT imaging system, including for example a gantry of a CT imaging system, a patient table of a CT imaging system, and so forth. It is also to be appreciated that the method may be used to evaluate performance metrics for other types of components in other types of medical imaging systems. For instance, the method may be used to evaluate performance metrics for components in projection X-ray imaging systems, MRI systems, ultrasound imaging systems, positron emission tomography, PET, imaging systems, single photon emission computed tomography, SPECT imaging systems, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.
As mentioned above, there remains a need to improve the acquisition of monitoring data for evaluating performance metrics of medical imaging systems.

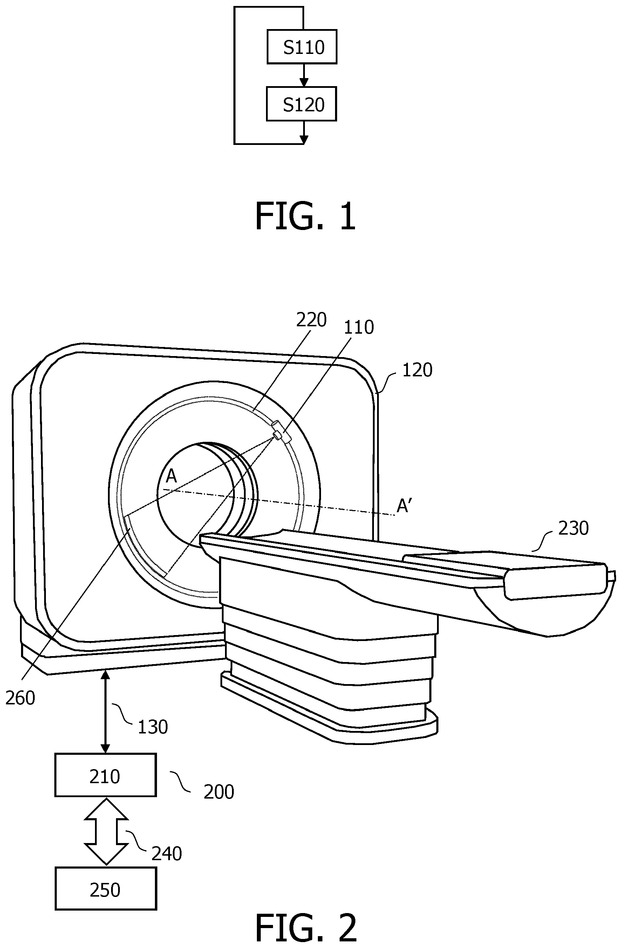

Fig. 1 is a flowchart illustrating an example of a computer-implemented method of evaluating a performance metric for a component of a medical imaging system, in accordance with some aspects of the present disclosure. Fig. 2 is a schematic diagram illustrating an example of a system 200 for evaluating a performance metric for a component 110 of a medical imaging system 120, in accordance with some aspects of the present disclosure. The system 200 includes one or more processors 210. It is noted that operations described in relation to the method illustrated in Fig. 1, may also be performed by the one or more processors 210 of the system 200 illustrated in Fig. 2. Likewise, operations described in relation to the one or more processors 210 of the system 200 illustrated in Fig. 2, may also be performed in the method described with reference to Fig. 1.

With reference to Fig. 1, the computer-implemented method of evaluating a performance metric for a component 110 of a medical imaging system 120, includes:
- acquiring S110 monitoring data 130 for the component 110 during a monitoring period 140₁;
- wherein the monitoring data 130 comprises values 150 of a set of monitored parameters 160, and wherein the values of the set of monitored parameters are acquired under a corresponding set of measurement conditions 170; and
- wherein the monitoring data 130 is acquired during a plurality of separate time intervals 180_{1..i} within the monitoring period 140₁, each time interval being prior to a performance of a diagnostic imaging scan 190 by the medical imaging system, or subsequent to a performance of a diagnostic imaging scan by the medical imaging system; and
- wherein the method further comprises:
   - calculating S 120 the value of the performance metric for the component 110 based on the values 150 of the set of monitored parameters 160 acquired during the monitoring period 140₁; and
   - repeating the acquiring S110, and the calculating S120, for monitoring data acquired during one or more subsequent monitoring periods 140₂, 140₃, and wherein the monitoring data acquired during the one or more subsequent monitoring periods is acquired using a revised set of monitored parameters 160', 160", or a revised set of measurement conditions 170', 170"; and
   - wherein the revised set of monitored parameters 160', 160", or the revised set of measurement conditions 170', 170", used to acquire the monitoring data during the one or more subsequent monitoring periods 140₂, 140₃ is determined based on a rate of change of a value 150 of one or more of the monitored parameters 160 during a preceding monitoring period 140₁.

In the above method, monitoring data is acquired during time intervals that are prior to a performance of a diagnostic imaging scan by the medical imaging system, or subsequent to a performance of a diagnostic imaging scan by the medical imaging system. The use of such time periods avoids interruptions to a diagnostic imaging scan. Moreover, the use of such time periods facilitates the acquisition of monitoring data under measurement conditions that are difficult, or even impossible, to provide during a diagnostic imaging scan due to their potential impact on the scan. Thus, it facilitates access to a wider range of measurement conditions. This wider range of measurement conditions can result in observations that are either not apparent, or entirely absent, from measurement data that is acquired data during a scan. Thus, it helps to elucidate subtle changes in the performance of a component.

In the above method, the revised set of monitored parameters, or the revised set of measurement conditions, that is used to acquire the monitoring data during the one or more subsequent monitoring periods, is determined based on a rate of change of a value of one or more of the monitored parameters during a preceding monitoring period. An insight of the inventors that is exploited in this operation, is that monitored parameters, or measurement conditions, that give rise to monitored parameter values that undergo relatively smaller changes over time have relatively less impact on the calculated value of the performance metric than monitored parameter values that undergo relatively larger changes over time. Revising the set of monitored parameters, or the revised set of measurement conditions, based on a rate of change of a value of one or more of the monitored parameters, facilitates the acquisition of data from monitored parameters, or under measurement conditions, that has a greater impact on the performance metric. It also helps to limit the data storage and transmission requirements of the monitoring data. For instance, monitored parameters that give rise to monitored parameter values that undergo relatively smaller changes over time, can be removed from the set, or measured at relatively larger time intervals. Similarly, measurement conditions that give rise to monitored parameter values that undergo relatively smaller changes over time, can be adjusted in order to provide monitored parameter values that undergo relatively larger changes over time. In other words, the method provides for the acquisition of monitoring data that has greater relevance to the performance metric. Thus, it improves the accuracy of the performance metric, and also alleviates the data storage requirements, and the communication bandwidth requirements, of storing, and transmitting, the monitoring data.

With reference to the method illustrated in Fig. 1, in the operation S110, monitoring data 130 is acquired for the component 110 during a monitoring period 140₁. The monitoring data 130 comprises values 150 of a set of monitored parameters 160, and the values of the set of monitored parameters are acquired under a corresponding set of measurement conditions 170.

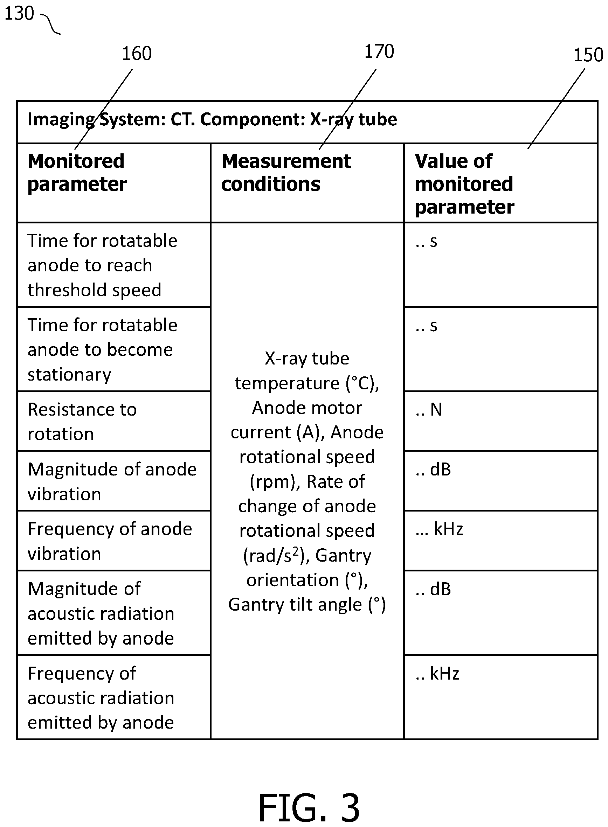

Fig. 3 illustrates an example of monitoring data 130 that is acquired for an X-ray tube of a CT imaging system, in accordance with some aspects of the present disclosure. The monitoring data 130 including values 150 for a set of monitored parameters 160 that are acquired under a corresponding set of measurement conditions 170. In the example illustrated in Fig. 3, the X-ray tube includes a rotatable anode, and the monitored parameters 160 include e.g. a time for the rotatable anode to reach a threshold speed, a time for the rotatable anode to become stationary, a resistance to rotation of the anode. The other monitored parameters 160 in the example illustrated in Fig. 3 relate to vibration of the anode during its rotation, and to acoustic radiation emitted by the anode during its rotation. Such parameters have been identified as useful parameters to monitor in order to evaluate performance metrics for the X-ray tube. For instance, over time, an increase in the time taken for the anode to reach a target threshold speed, and a reduction in the time taken for the anode to become stationary, have both been associated with increased wear and tear in the anode bearing. Similarly, the anode's resistance to rotation, and the magnitude of vibration of the anode, and the magnitude of acoustic radiation generated by the anode, have also been associated with increased wear and tear in the anode bearing. Similarly, such changes in these parameters have also been found to be associated with an increased likelihood of faults with the X-ray tube.

The monitored parameters 160 illustrated in Fig. 3 are acquired under corresponding measurement conditions 170. In the example illustrated in Fig. 3, these include the X-ray tube temperature, the anode motor current, the anode rotational speed, and so forth. As may be appreciated, the total parameter space of the monitored parameters 160, over the complete range of measurement condition 170 for each monitored parameter, can be very large. Consequently, it may be impractical to acquire values of monitored parameters 150 for the monitored parameters 160, over the complete range of each measurement condition. Thus, monitoring data for monitored parameters is often only acquired under a fixed set of measurement conditions.

By contrast, wear and tear, and also faults associated with the X-ray tube, can give rise to effects that are only measurable via the monitored parameters 160 under specific measurement conditions 170. For instance, vibration associated with the rotation of the anode of the X-ray tube might only be measurable within a narrow range of the anode's rotational speed. Also, the vibration might also only be significant at specific vibrational frequencies. Over time, the range of anode rotational speed at which the vibration is measurable, and also the frequencies of the vibration, may change. Consequently, monitoring data that is acquired for a component under fixed measurement conditions can miss valuable information that is relevant to evaluating the performance of the component.

In general, the values 150 of the set of monitored parameters 160 that are acquired in the operation S 110 are provided by corresponding sensors. Sensors may likewise be used to monitor the measurement conditions 170. The sensors may be disposed within, or proximal to, the medical imaging system 120. For instance, sensors may be disposed within a housing of the CT imaging system 120 illustrated in Fig. 1, or they may be disposed within a room in which the CT imaging system 120 is located. Various sensors may be employed for this purpose, including for example, rotation sensors, vibration sensors (e.g. an accelerometer or a gyroscope), acoustic sensors, radio frequency, RF, sensors, current sensors, voltage sensors, temperature sensors, and so forth.

In one example, the component 110 of the medical imaging system 120 comprises an X-ray tube of a projection X-ray imaging system, or a CT imaging system, and the set of monitored parameters 160 represents one or more of:
- a time taken for a rotatable anode of the X-ray tube to accelerate to a threshold speed;
- a time taken for the rotatable anode of the X-ray tube to decelerate to a stationary speed;
- a resistance to rotation of a rotatable anode of the X-ray tube; and
- a magnitude and/ or a frequency of vibration of an anode of the X-ray tube;
- a magnitude and/ or a frequency of acoustic radiation emitted by the anode of the X-ray tube.

In this example, the corresponding set of measurement conditions 170 comprises one or more of:
- a temperature of the X-ray tube;
- a magnitude of a current supplied to a motor configured to rotate an anode of the X-ray tube;
- a rotational speed of a rotatable anode of the X-ray tube;
- a rate of change of a rotational speed of a rotatable anode of the X-ray tube;
- an orientation, or a tilt angle, of a gantry 220 of the projection X-ray imaging system, or the CT imaging system; and
- a rotational speed of a gantry 220 of the CT imaging system.

In other examples, the monitoring data 130 that is acquired in the operation S110 may include the values of other monitored parameters, and these may be acquired under different measurement conditions, and using corresponding sensors. Thus, in another example, the component 110 of the medical imaging system 120 comprises a gantry 220 of a projection X-ray imaging system, or a CT imaging system, and the set of monitored parameters 160 comprises one or more of:
- a magnitude and/or a frequency of vibration of the gantry 220; and
- a magnitude and/ or a frequency of acoustic radiation emitted by the gantry 220.

In this example, the corresponding set of measurement conditions 170 comprises one or more of:
- a rotational speed of the gantry 220;
- a rotational speed profile of the gantry 220;
- a temperature of the gantry 220;
- an angular position of the gantry 220 at which the parameter is measured; and
- a rate of change of a rotational speed of the gantry 220.

In another example, the component 110 of the medical imaging system 120 comprises a coil of a magnetic resonance imaging, MRI, system, and wherein the set of monitored parameters 160 comprises one or more of:
- a magnitude and/ or a frequency of vibration of the coil; and
- a magnitude and/ or a frequency of acoustic radiation emitted by the coil;

In this example, the corresponding set of measurement conditions 170 comprises one or more of:
- a temperature of the MRI coil;
- a pattern of currents applied to the MRI coil;
- a magnitude of a current applied to the MRI coil;
- an activation status of the MRI cold head.

In another example, the component 110 of the medical imaging system 120 comprises a patient table 230, and wherein the set of monitored parameters 160 comprises one or more of:
- a magnitude and/or a frequency of vibration of the patient table; and
- a magnitude and/ or a frequency of acoustic radiation emitted by the patient table;

In this example, the corresponding set of measurement conditions 170 comprises one or more of:
- a horizontal translational speed of the patient table 230;
- a vertical speed of the patient table 230;
- a temperature of the patient table 230;
- a movement profile of the patient table 230.

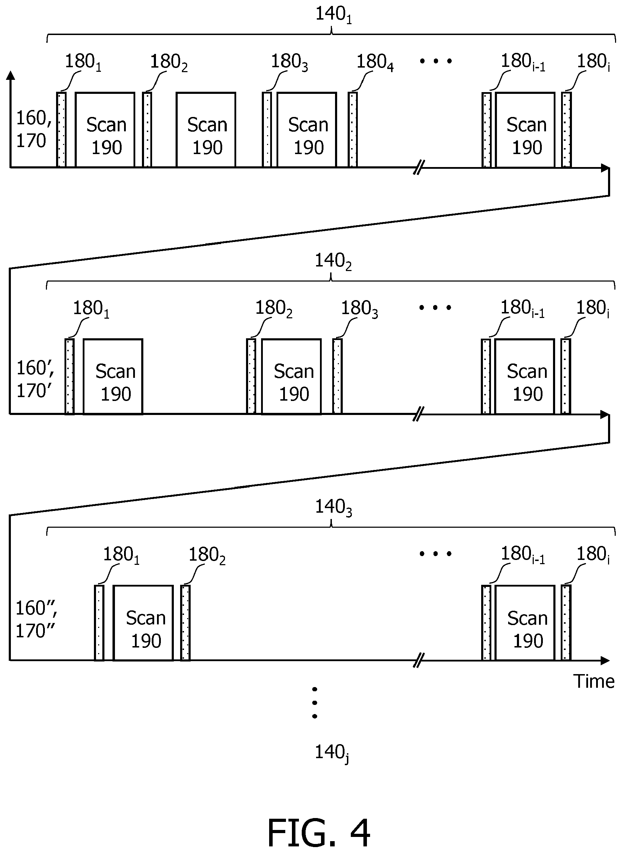

Returning to the method illustrated in Fig. 1, in the operation S110, the monitoring data 130 that is acquired for the component 110 is acquired during a monitoring period 140₁. The monitoring data 130 is acquired during a plurality of separate time intervals 180_{1..i} within the monitoring period 140₁. Each time interval is prior to a performance of a diagnostic imaging scan 190 by the medical imaging system, or subsequent to a performance of a diagnostic imaging scan by the medical imaging system. Fig. 4 is an example illustrating the acquisition of monitoring data during a plurality of separate time intervals 180_{1..i} within a monitoring period 140₁, and wherein each time interval is prior to a performance of a diagnostic imaging scan 190 by a medical imaging system, or subsequent to a performance of a diagnostic imaging scan by the medical imaging system, in accordance with some aspects of the present disclosure. As mentioned above, by avoiding the time during which diagnostic scans are performed, interruptions to diagnostic imaging scans are avoided.

In some examples, the set of measurement conditions 170 includes measurement conditions that are unobtainable during a diagnostic imaging scan 190 by the medical imaging system. With reference to the example illustrated in Fig. 3, a diagnostic imaging scan 190 by a projection X-ray, or CT, imaging system, is typically performed with the X-ray tube in a warmed-up state and with the anode rotated at a constant rotational speed. Thus, measurement data that is acquired during a scan is typically limited to a narrow range of measurement conditions such as X-ray tube temperature, anode motor current, anode rotational speed, and rate of change of anode rotational speed. Since the method described with reference to Fig. 1 includes acquiring monitoring data for a component during a monitoring period that is outside of the time of performing a diagnostic imaging scan by the medical imaging system, measurement conditions that are unobtainable during a diagnostic imaging scan may be used to acquire the monitoring data without interrupting the scan. The use of measurement conditions that are unobtainable during a diagnostic imaging scan 190 facilitates the acquisition of monitoring data from a wider region of the parameter space than is available by solely acquiring monitoring data during a scan. The measurement conditions in this parameter space may give rise to parameter values that vary significantly over time. Consequently, the use of this wider region of the parameter space facilitates the monitoring of performance metrics that are unobservable or only weakly observable, from analysing measurement data that is acquired during a scan.

The monitoring periods 140 may be set in various ways. For example, a monitoring period 140 may be set to a specified time, or to specified number of scans. For instance, a monitoring period may be set to a specified number of hours, days, weeks, or months, or it may be set to a specified number of scans, or more specifically to a specified number of scans of the same type. For instance, in the example of a diagnostic imaging scan by a CT imaging system, a monitoring period 140 may be set to a specified number of scans of a specified body region, such as the brain, or it may be set to a specified number of scans performed at a specified X-ray energy level, e.g. at 120 keV.

The time intervals 180_{1..i} during which the monitoring data 130 is acquired, are prior to a performance of a diagnostic imaging scan 190 by a medical imaging system, or subsequent to a performance of a diagnostic imaging scan by the medical imaging system. In other words, the time intervals 180_{1..i} are outside of the time during which diagnostic imaging scans are performed. In one example, the time intervals 180_{1..i} occur during a so-called "warm-up period", or "ramp-up period", when the medical imaging system is started-up, or switched out of a standby mode in preparation for a diagnostic imaging scan. With reference to the example in which the component is an X-ray tube of a projection X-ray imaging system, or a CT imaging system, the time intervals 180_{1..i} occur prior to a performance of a diagnostic imaging scan 190 by the medical imaging system, and during a period in which the rotatable anode of the X-ray tube is accelerating. This time period facilitates the use of a variety of different measurement conditions 150, including for example different X-ray tube temperatures, different anode motor currents, and different anode rotational speeds. Consequently it provides access to various parts of the ranges of these measurement conditions that may be unobtainable during a diagnostic imaging scan. In another example, the time intervals 180_{1..i} occur subsequent to a performance of a diagnostic imaging scan 190 by the medical imaging system, and during a period in which the rotatable anode of the X-ray tube is decelerating. This may be referred to as a "cool-down period" when the medical imaging system is prepared for entering a standby mode, or a switched-off mode. With reference to the example in which the component is an X-ray tube of a CT imaging system, the time intervals may occur during a period in which the anode is decelerating to a stationary position. During this period, the anode may undergo a so-called "free-run" in which the anode is allowed to come to the stationary position under its own accord, or the anode may undergo a so-called "active breaking" procedure wherein the anode is actively brought to a halt. This time period likewise provides access to a variety of different measurement conditions 150 that may be unobtainable during a diagnostic imaging scan.

Returning to the method illustrated in Fig. 1, in the operation S120, the value of a performance metric is calculated for the component 110 based on the values 150 of the set of monitored parameters 160 acquired during the monitoring period 140₁.

The calculation of various performance metrics is contemplated in the operation S120. In general, the performance metric may represent one or more of: an amount of degradation associated with the component, an expected fault with the component, and an expected remaining lifetime of the component. The performance metrics may be evaluated using empirically-determined relationships, or algorithms, including for example algorithms that are trained using artificial intelligence techniques.

In the example of the component being a bearing of an anode of an X-ray tube, the performance metric may for instance be evaluated using an empirically-determined relationship between vibration, or acoustic signals generated by the bearing, and the performance metric. The empirical relationship may be generated by analysing historical vibration data and recording the corresponding performance metrics in representative components. Subsequently, the empirical relationship may be used to evaluate the performance metric for newly-acquired monitored parameters. For instance, in the example of the performance metric representing an amount of degradation, or a fault with the bearing, spectral analysis techniques described in a document by Randall, R. B., et al. "Rolling element bearing diagnostics - A tutorial", Mechanical Systems and Signal Processing, 25, (2011) pages 485-520, may be used. This document describes spectral analysis techniques such as "envelope analysis" for analysing vibration signals in order to identify different ball bearing failure modes, and "spectral kurtosis" that may be used to identify frequencies that are indicative of impulsive fault characteristics. The spectral kurtosis value has been found to correlate with bearing wear, and may therefore be used as a performance metric for the degradation of the bearing. A measure of the expected remaining lifetime of a component may be determined from such wear metrics by setting a threshold value to the amount of wear corresponding to an expected failure, or by performing a statistical analysis on the faults.

Similarly, statistical methods that are based on pattern recognition may be used to predict an amount of degradation, an expected fault, or an expected remaining lifetime of the component, from historical monitoring data. For instance, historical monitoring data may be acquired from components that have been in-service, together with corresponding performance data representing e.g. the remaining lifetime, the amount of degradation, and specific faults that have been observed. Patterns that are characteristic of specific faults, may then be extracted from the historical monitoring data. Subsequently, the value of the performance metric, e.g. the severity of a specific fault, may be determined based on a magnitude of a correlation between newly-acquired monitoring data, and the characteristic patterns from the historical monitoring data. This analysis may also be performed by training an AI algorithm to predict the performance metric based on the monitoring data, using training data that includes the historical monitoring data and ground truth data representing the performance metric.

In some examples, the analysis is performed subsequent to performing a data cleaning operation on the acquired monitoring data 130. The data cleaning operation involves subtracting interference signals from the acquired monitoring data that arise from elements in the medical imaging system other than the component 110. The data cleaning operation may be performed by e.g. filtering the acquired monitoring data, or subtracting signal patterns that represent known interference signals, from the acquired monitoring data. For instance, interference signals arising from the vibration of the rotating gantry of a CT imaging system, or a pump associated with the cooling system, and which are overlaid onto desired signals representing ball bearing vibration in an X-ray tube anode, may be removed during the data cleaning operation in this manner. Such interference signals may alternatively be reduced, or even eliminated, by suitably adjusting the measurement conditions under which the desired signals are acquired. For instance, interference signals arising from the vibration of the rotating gantry of a CT imaging system may be eliminated by measuring signals representing ball bearing vibration in an X-ray tube anode when the gantry is static.

Returning to the method illustrated in Fig. 1, after calculating the value of the performance metric for the component 110 based on the values 150 of the set of monitored parameters 160 acquired during the monitoring period 140₁, the method continues by repeating the acquiring S110, and the calculating S120, for monitoring data acquired during one or more subsequent monitoring periods 140₂, 140₃. This operation is illustrated in the central and lower portions of Fig. 4, for the time periods 140₂, and 140₃, respectively. The one or more subsequent monitoring periods 140₂, 140₃ may be defined in a similar manner to the monitoring period 140₁. In other words, the subsequent monitoring period(s) may have the same, or a similar, duration, or they may correspond to the same, or a similar, number of scans, and so forth, as the monitoring period 140₁.

The monitoring data acquired during the one or more subsequent monitoring periods 140₂, 140₃, is acquired using a revised set of monitored parameters 160', 160", or a revised set of measurement conditions 170', 170". The revised set of monitored parameters 160', 160", or the revised set of measurement conditions 170', 170", used to acquire the monitoring data during the one or more subsequent monitoring periods 140₂, 140₃ is determined based on a rate of change of a value 150 of one or more of the monitored parameters 160 during a preceding monitoring period 140₁. Thus, in this operation, the rate of change of the value 150 of one or more of the monitored parameters 160, is determined.

As described above, an insight of the inventors that is exploited in this operation, is that monitored parameters, or measurement conditions, that give rise to monitored parameter values that undergo relatively smaller changes over time have relatively less impact on the calculated value of the performance metric than monitored parameter values that undergo relatively larger changes over time. Revising the set of monitored parameters, or the revised set of measurement conditions, based on a rate of change of a value of one or more of the monitored parameters, facilitates the acquisition of data from monitored parameters, or under measurement conditions, that has a greater impact on the performance metric. It also helps to limit the data storage requirements of the monitoring data. For instance, monitored parameters that give rise to monitored parameter values that undergo relatively smaller changes over time, can be removed from the set, or measured at relatively larger time intervals. Similarly, measurement conditions that give rise to monitored parameter values that undergo relatively smaller changes over time, can be adjusted in order to provide monitored parameter values that undergo relatively larger changes over time. In other words, the method provides for the acquisition of monitoring data that has greater relevance to the performance metric. This improves the accuracy of the performance metric, and also alleviates the data storage requirements, and the communication bandwidth requirements, of storing, and transmitting, the monitoring data.

Thus, in general, it is contemplated to remove monitored parameters from the set of monitored parameters 160 and/or to adjust measurement conditions in the set of measurement conditions 170, in response to the rate of change of the value 150 of monitored parameters 160. In one example, the revised set of monitored parameters 160', 160" is determined by removing one or more of the monitored parameters from the set of monitored parameters 160 based on the rate of change of the value of the corresponding one or more of the monitored parameters during the preceding monitoring period 140₁. In another example, the revised set of measurement conditions 170', 170", is determined by adjusting one or more of the measurement conditions in the set of measurement conditions 170, based on the rate of change of the value of the corresponding one or more of the monitored parameters during the preceding monitoring period 140₁. A combination of both of these approaches may also be used. For instance, some parameters may be removed from the set of monitored parameters, and the measurement conditions of other monitored parameters may be adjusted.

By way of an example, many mechanical components of medical imaging systems, e.g. the gantry rotation system of a CT or projection X-ray imaging system, a rotatable anode of an X-ray tube, a patient table, have characteristic vibration frequencies that are useful to measure during operation, and which can be used to evaluate performance metrics. Thus, it may be decided to initially acquire monitoring data representing the magnitude of vibration at each of these frequencies during a monitoring period 140₁, under a corresponding set of measurement conditions, e.g. a specific rotational speed of the gantry, or anode, a specific X-ray tube temperature, and so forth. Having evaluated the rates of change of the magnitudes of vibration at each of these frequencies over the monitoring period 140₁, it may be found that the magnitudes of some of these vibrational frequencies vary negligibly over time, and consequently it may be concluded that they convey relatively little information about the performance of the component. Consequently, it may be decided to remove from the set of monitored parameters, specific vibrational frequencies for which the corresponding magnitude underwent relatively little variation over the monitoring period 140₁, and to consequently acquire monitoring data during the one or more subsequent monitoring periods 140₂, 140₃, from a reduced set of frequencies. Alternatively or additionally, the measurement conditions of the monitored frequencies that were found to undergo relatively smaller changes over time, may be adjusted, for example by changing the rotational speed of the gantry, or anode, or by changing the X-ray tube temperature, and so forth, and acquiring monitoring data during the one or more subsequent monitoring periods 140₂, 140₃, using different values for these measurement conditions. In so doing, over time, a set of monitored parameters 160, and a corresponding set of measurement conditions 170, are identified that give rise to monitored parameters with relatively higher rates of change in their values, and which consequently have relatively higher relevance to the calculated value of the performance metric.

In one example, the operation of removing one or more of the monitored parameters from the set of monitored parameters 160, comprises:
- comparing the rate of change of the value of the one or more of the monitored parameters 150 during the preceding monitoring period 140₁, with a corresponding threshold value; and
- removing from the set of monitored parameters 160, one or more of the monitored parameters having a rate of change that fails to exceed the corresponding threshold value.

In this example, the threshold value may be set to any desired rate of change. For instance, the threshold value may be set for each of the monitored parameters based on empirically-determined rates for correctly-functioning components. The threshold values may be changed over time in order to ensure that some, but not all, of the parameters are removed, or retained, at the end of each monitoring period. In this way, the threshold value may set so as to provide a balance between the benefit of maintaining a stable set of monitored parameters, i.e. the provision of a consistent set of data from which to measure the performance metric, and the benefit of reducing data storage requirements that is obtained by removing parameters that are deemed to have low relevance.

In a related example, the revised set of monitored parameters 160', 160" is determined by removing one or more of the monitored parameters from the set of monitored parameters 160, based on the rate of change of the value of the corresponding one or more of the monitored parameters during the preceding monitoring period 140₁, and wherein the method described with reference to Fig. 1 comprises:
- including one or more additional parameters in the set of monitored parameters 160, and wherein the one or more additional parameters included in the set of monitored parameters comprises one or more of:
- a parameter identified as having a rate of change exceeding a corresponding threshold value for a corresponding component in a different medical imaging system; and
- a randomly-selected parameter.

Thus, in this example, a removed parameter is replaced by another parameter. Parameters that have been identified as having a rate of change exceeding a corresponding threshold value for a corresponding component in a different medical imaging system, may be identified by using the method described with reference to Fig. 1 in the different imaging system, and selecting parameters that have remained in the set of monitored parameters for multiple monitoring periods. The imaging system, and the different imaging systems may form part of a fleet of imaging systems. Thus, the parameter identified as having a rate of change exceeding a corresponding threshold value for a corresponding component in a different medical imaging system may originate from a different imaging system in the fleet. This sharing of parameters between the different imaging systems in the fleet may be referred-to as "federated learning", and provides relatively newer medical imaging systems in the fleet with the most relevant monitored parameters for a component, based on the experience gained by relatively older medical imaging systems. For example, if a new X-ray tube of a CT imaging system is placed into service, the method may facilitate a monitoring of the new X-ray tube with the monitored parameters from relatively older X-ray tubes in the fleet, and for which a corpus of monitoring data has been acquired. Federated learning can be used for similar systems within a fleet, i.e. for identical systems within a fleet, or for different versions of systems having identical or similar components.

In the case that a randomly-selected parameter is used to replace a parameter that has been removed, the randomly-selected parameter might identify a region of the parameter space that gives a high rate of variation over time. Thus, it might lead to the identification of a parameter that is unexpectedly relevant to the monitoring of the component's performance.

In another related example, the operation of including one or more additional parameters in the set of monitored parameters 160, is performed during the monitoring period 140₁. The one or more additional parameters may be included at a start of the monitoring period, or they may be included at another time during the monitoring period. The rates of change of the parameters that have been acquired over the monitoring period 140₁, including those of the included monitored parameters, are then analysed at the end of the monitoring period. One or more parameters are then removed from the set of monitored parameters 160, based on the rate of change of their values. In this way, if a newly-included parameter turns out to have values with a lower rate of change than existing monitored parameters, the newly-included monitoring parameter may be removed from the set. This allows experiments to be performed on the composition of the set of monitored parameters without the risk of gaps in the data for existing monitored parameters.

In another example, the monitoring data acquired during the one or more subsequent monitoring periods is acquired using a revised set of measurement conditions 170', 170". In this example, the revised set of measurement conditions 170', 170" is determined by iteratively adjusting one or more of the measurement conditions in the set of measurement conditions 170, in response to the rate of change of the value of the corresponding one or more of the monitored parameters 160 during the preceding monitoring period 140₁. In this example, the measurement conditions may be iteratively adjusted by employing an optimisation technique, such as gradient ascent, or the simplex method, to find measurement conditions that provide optimised rates of change of the values 150 of the monitored parameters 160.

In another example, a value of a weighted sum of the rates of change of a plurality of values of the one or more of the monitored parameters during a preceding monitoring period 140₁, is calculated. In this example, the revised set of monitored parameters 160', 160", or the revised set of measurement conditions 170', 170", used to acquire the monitoring data during the one or more subsequent monitoring periods 140₂, 140₃ is determined based further on the value of the weighted sum. In this example, weights are applied to the rates of change of the values of the monitored parameters. In some examples, the weights may be equal, whereas in other examples, different weights may be applied to the individual rates in order to set the relative contributions of the individual rates to the value of the weighted sum. By using the value of the weighted sum to determine the revised set of monitored parameters 160', 160", or the revised set of measurement conditions 170', 170", it may for instance be decided that overall, the current set of monitored parameters has sufficient variation over time to justify maintaining the current set of monitored parameters, thereby enabling the acquisition of a set of data over multiple time intervals with a stable set of measurement parameters, or measurement conditions.

In another example, the method described with reference to Fig. 1 includes:
- receiving an initial set of monitored parameters 160, and a corresponding initial set of measurement conditions 170, wherein the initial set of monitored parameters have been identified as having a rate of change exceeding a corresponding threshold value, under the initial set of measurement conditions, in a corresponding component in a different medical imaging system; and
- wherein the acquiring S210 monitoring data 130, is performed using the initial set of monitored parameters, and under the initial set of measurement conditions.

In this example, the initial monitored parameters 160, and the corresponding initial set of measurement conditions 170 may be identified by using the method described with reference to Fig. 1 in the different imaging system, and selecting parameters that have remained in the set of monitored parameters for multiple monitoring periods. The imaging system, and the different imaging system may form part of a fleet of imaging systems. As described above, this sharing of information between the different imaging systems in the fleet provides relatively newer medical imaging systems with the most relevant monitored parameters for a component, based on the experience gained by relatively older medical imaging systems. For example, if a new X-ray tube of a CT imaging system is placed into service, the method may facilitate a monitoring of the new X-ray tube with the monitored parameters from relatively older X-ray tubes, and for which a corpus of monitoring data has been acquired.

In another example, the method described with reference to Fig. 1 includes transmitting the acquired monitoring data 130 over a communication channel 240 to a remote processing device 250. This example is illustrated in Fig. 2. In this example, the calculating operation S120 is performed by the remote processing device 250. Performing the calculating operation in the remote processing device 250 alleviates the processing requirements of executing the method locally with respect to the medical imaging system 120. Since, in this example the acquired monitoring data 130 is transmitted, and since the amount of acquired data has been controlled in accordance with the method described with reference to Fig. 1, the bandwidth requirements of the communication channel 240 over which the monitoring data 130 is transmitted, are alleviated.

In another example, the method described with reference to Fig. 1 also includes performing one or more of the following based on the value of the performance metric:
- generating a notification to a user;
- triggering a change in operation of the component 110; and
- generating a service call to schedule a maintenance operation on the component 110.

In this example, the notification to a user may for instance indicate that the value of the performance metric meets a threshold condition. For instance, it may indicate that the remaining lifetime of the component is below a threshold lifetime value. Likewise the notification may indicate that the total amount of degradation of a component has exceeded a threshold level. In so doing, the user may be alerted to the need to replace, repair, or perform another service action on the component.

The triggering of a change in operation of the component 110 in this example may for instance switch the component into a de-activated, or a relatively lower power mode of operation in order to prevent damage to the component. The triggering may be performed subject to a user confirmation of a prompt to change the operation of the component 110. In the example of the component being an X-ray tube, the change in the operation of the component may include reducing an energy level of the X-ray emitted by the X-ray tube, or reducing an amount of power applied to the X-ray tube.

The generation of a service call to schedule a maintenance operation on the component 110 in this example, has the effect of reducing the likelihood of damage to the component through its continued use.

In another example, a computer program product, is provided. The computer program product comprising instructions which when executed by one or more processors 210, cause the one or more processors to carry out a method of evaluating a performance metric for a component 110 of a medical imaging system 120. The method comprises:
- acquiring S110 monitoring data 130 for the component 110 during a monitoring period 140₁;
- wherein the monitoring data 130 comprises values 150 of a set of monitored parameters 160, and wherein the values of the set of monitored parameters are acquired under a corresponding set of measurement conditions 170; and
- wherein the monitoring data 130 is acquired during a plurality of separate time intervals 180_{1..i} within the monitoring period 140₁, each time interval being prior to a performance of a diagnostic imaging scan 190 by the medical imaging system, or subsequent to a performance of a diagnostic imaging scan by the medical imaging system; and
- wherein the method further comprises:
- calculating S120 the value of the performance metric for the component 110 based on the values 150 of the set of monitored parameters 160 acquired during the monitoring period 140₁; and
- repeating the acquiring S110, and the calculating S120, for monitoring data acquired during one or more subsequent monitoring periods 140₂, 140₃, and wherein the monitoring data acquired during the one or more subsequent monitoring periods is acquired using a revised set of monitored parameters 160', 160", or a revised set of measurement conditions 170', 170"; and
- wherein the revised set of monitored parameters 160', 160", or the revised set of measurement conditions 170', 170", used to acquire the monitoring data during the one or more subsequent monitoring periods 140₂, 140₃ is determined based on a rate of change of a value 150 of one or more of the monitored parameters 160 during a preceding monitoring period 140₁.

In another example, a system 200 for evaluating a performance metric for a component 110 of a medical imaging system 120, is provided. The system comprises one or more processors 210 configured to:
- acquire S110 monitoring data 130 for the component 110 during a monitoring period 140₁;
- wherein the monitoring data 130 comprises values 150 of a set of monitored parameters 160, and wherein the values of the set of monitored parameters are acquired under a corresponding set of measurement conditions 170; and
- wherein the monitoring data 130 is acquired during a plurality of separate time intervals 180_{1..i} within the monitoring period 140₁, each time interval being prior to a performance of a diagnostic imaging scan 190 by the medical imaging system, or subsequent to a performance of a diagnostic imaging scan by the medical imaging system.

The one or more processors 210 are further configured to:
- calculate S120 the value of the performance metric for the component 110 based on the values 150 of the set of monitored parameters 160 acquired during the monitoring period 140₁; and
- repeat the acquiring S110, and the calculating S120, for monitoring data acquired during one or more subsequent monitoring periods 140₂, 140₃, and wherein the monitoring data acquired during the one or more subsequent monitoring periods is acquired using a revised set of monitored parameters 160', 160", or a revised set of measurement conditions 170', 170"; and
- wherein the revised set of monitored parameters 160', 160", or the revised set of measurement conditions 170', 170", used to acquire the monitoring data during the one or more subsequent monitoring periods 140₂, 140₃ is determined based on a rate of change of a value 150 of one or more of the monitored parameters 160 during a preceding monitoring period 140₁.

An example of the system 200 is illustrated in Fig. 2. It is noted that the system 200 may also include one or more sensors for generating the monitoring data 130, examples of which are described above. It is noted that the system 200 may be provided as part of a medical imaging system 120, such as the CT imaging system illustrated in Fig. 2, for example.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of evaluating a performance metric for a component (110) of a medical imaging system (120), the method comprising:
acquiring (S110) monitoring data (130) for the component (110) during a monitoring period (140₁);
wherein the monitoring data (130) comprises values (150) of a set of monitored parameters (160), and wherein the values of the set of monitored parameters are acquired under a corresponding set of measurement conditions (170); and
wherein the monitoring data (130) is acquired during a plurality of separate time intervals (180_{1..i}) within the monitoring period (140₁), each time interval being prior to a performance of a diagnostic imaging scan (190) by the medical imaging system, or subsequent to a performance of a diagnostic imaging scan by the medical imaging system; and
wherein the method further comprises:
calculating (S120) the value of the performance metric for the component (110) based on the values (150) of the set of monitored parameters (160) acquired during the monitoring period (140₁); and
repeating the acquiring (S110), and the calculating (S120), for monitoring data acquired during one or more subsequent monitoring periods (140₂, 140₃), and wherein the monitoring data acquired during the one or more subsequent monitoring periods is acquired using a revised set of monitored parameters (160', 160"), or a revised set of measurement conditions (170', 170"); and
wherein the revised set of monitored parameters (160', 160"), or the revised set of measurement conditions (170', 170"), used to acquire the monitoring data during the one or more subsequent monitoring periods (140₂, 140₃) is determined based on a rate of change of a value (150) of one or more of the monitored parameters (160) during a preceding monitoring period (140₁).

2. The computer-implemented method according to claim 1, wherein the revised set of monitored parameters (160', 160") is determined by removing one or more of the monitored parameters from the set of monitored parameters (160) based on the rate of change of the value of the corresponding one or more of the monitored parameters during the preceding monitoring period (140₁); and/ or
wherein the revised set of measurement conditions (170', 170"), is determined by adjusting one or more of the measurement conditions in the set of measurement conditions (170), based on the rate of change of the value of the corresponding one or more of the monitored parameters during the preceding monitoring period (140₁).

3. The computer-implemented method according to claim 2, wherein the removing one or more of the monitored parameters from the set of monitored parameters (160), comprises:
comparing the rate of change of the value of the one or more of the monitored parameters (150) during the preceding monitoring period (140₁), with a corresponding threshold value; and
removing from the set of monitored parameters (160), one or more of the monitored parameters having a rate of change that fails to exceed the corresponding threshold value.

4. The computer-implemented method according to claim 2 or claim 3, wherein the revised set of monitored parameters (160', 160") is determined by removing one or more of the monitored parameters from the set of monitored parameters (160), based on the rate of change of the value of the corresponding one or more of the monitored parameters during the preceding monitoring period (140₁); and
wherein the method further comprises:
including one or more additional parameters in the set of monitored parameters (160), and wherein the one or more additional parameters included in the set of monitored parameters comprises one or more of:
a parameter identified as having a rate of change exceeding a corresponding threshold value for a corresponding component in a different medical imaging system; and
a randomly-selected parameter.

5. The computer-implemented method according to claim 1, wherein the revised set of measurement conditions (170', 170") is determined by iteratively adjusting one or more of the measurement conditions in the set of measurement conditions (170), in response to the rate of change of the value of the corresponding one or more of the monitored parameters (160) during the preceding monitoring period (140₁).

6. The computer-implemented method according to claim 1, wherein the method further comprises:
calculating a value of a weighted sum of the rates of change of a plurality of values of the one or more of the monitored parameters during a preceding monitoring period (140₁); and
wherein the revised set of monitored parameters (160', 160"), or the revised set of measurement conditions (170', 170"), used to acquire the monitoring data during the one or more subsequent monitoring periods (140₂, 140₃) is determined based on a contribution of each parameter to the value of the weighted sum.

7. The computer-implemented method according to claim 1, wherein the component (110) of the medical imaging system (120) comprises an X-ray tube of a projection X-ray imaging system, or a CT imaging system, and wherein the set of monitored parameters (160) represents one or more of:
a time taken for a rotatable anode of the X-ray tube to accelerate to a threshold speed;
a time taken for the rotatable anode of the X-ray tube to decelerate to a stationary speed;
a resistance to rotation of a rotatable anode of the X-ray tube; and
a magnitude and/ or a frequency of vibration of an anode of the X-ray tube;
a magnitude and/ or a frequency of acoustic radiation emitted by the anode of the X-ray tube;
and wherein the corresponding set of measurement conditions (170) comprises one or more of:
a temperature of the X-ray tube; a magnitude of a current supplied to a motor configured to rotate an anode of the X-ray tube;
a rotational speed of a rotatable anode of the X-ray tube; a rate of change of a rotational speed of a rotatable anode of the X-ray tube;
an orientation, or a tilt angle, of a gantry (220) of the projection X-ray imaging system, or the CT imaging system; and
a rotational speed of a gantry (220) of the CT imaging system.

8. The computer-implemented method according to claim 1, wherein the component (110) of the medical imaging system (120) comprises a gantry (220) of a projection X-ray imaging system, or a CT imaging system, and wherein the set of monitored parameters (160) comprises one or more of:
a magnitude and/or a frequency of vibration of the gantry (220); and
a magnitude and/ or a frequency of acoustic radiation emitted by the gantry (220);
and wherein the corresponding set of measurement conditions (170) comprises one or more of:
a rotational speed of the gantry (220);
a rotational speed profile of the gantry (220);
a temperature of the gantry (220);
an angular position of the gantry (220) at which the parameter is measured; and
a rate of change of a rotational speed of the gantry (220).

9. The computer-implemented method according to claim 1, wherein the component (110) of the medical imaging system (120) comprises a coil of a magnetic resonance imaging, MRI, system, and wherein the set of monitored parameters (160) comprises one or more of:
a magnitude and/ or a frequency of vibration of the coil; and
a magnitude and/ or a frequency of acoustic radiation emitted by the coil;
and wherein the corresponding set of measurement conditions (170) comprises one or more of:
a temperature of the MRI coil;
a pattern of currents applied to the MRI coil;
a magnitude of a current applied to the MRI coil;
an activation status of the MRI cold head.

10. The computer-implemented method according to claim 1, wherein the component (110) of the medical imaging system (120) comprises a patient table (230), and wherein the set of monitored parameters (160) comprises one or more of:
a magnitude and/or a frequency of vibration of the patient table; and
a magnitude and/ or a frequency of acoustic radiation emitted by the patient table;
and wherein the corresponding set of measurement conditions (170) comprises one or more of:
a horizontal translational speed of the patient table (230);
a vertical speed of the patient table (230);
a temperature of the patient table (230);
a movement profile of the patient table (230).

11. The computer-implemented method according to claim 1, wherein the method further comprises:
receiving an initial set of monitored parameters (160), and a corresponding initial set of measurement conditions (170), wherein the initial set of monitored parameters have been identified as having a rate of change exceeding a corresponding threshold value, under the initial set of measurement conditions, in a corresponding component in a different medical imaging system; and
wherein the acquiring (S210) monitoring data (130), is performed using the initial set of monitored parameters, and under the initial set of measurement conditions.

12. The computer-implemented method according to any previous claim, wherein the component (110) comprises an X-ray tube of a projection X-ray imaging system, or a CT imaging system (120); and wherein the time interval (180) prior to a performance of a diagnostic imaging scan (190) by the medical imaging system corresponds to a time interval during which a rotatable anode of the X-ray tube is accelerating and/or wherein the time interval (180) subsequent to a performance of a diagnostic imaging scan (190) by the medical imaging system corresponds to a time interval during which the rotatable anode of the X-ray tube is decelerating.

13. The computer-implemented method according to any previous claim, wherein the performance metric represents one or more of:
an amount of degradation associated with the component;
a potential fault with the component; and
an expected remaining lifetime of the component.

14. The computer-implemented method according to any previous claim, wherein the method further comprises transmitting the acquired monitoring data (130) over a communication channel (240) to a remote processing device (250); and
wherein the calculating (S120) is performed by the remote processing device (250).

15. The computer-implemented method according to any previous claim, wherein the method further comprises performing one or more of the following based on the value of the performance metric:
generating a notification to a user;
triggering a change in operation of the component (110); and
generating a service call to schedule a maintenance operation on the component (110).
